# EUROPEAN PATENT APPLICATION

(11) **EP 1 952 829 A2**
(43) Date of publication of application: **06.08.2008**
(21) Application number: 08006101.3
(22) Date of filing: 01.12.2004
(51) Int. Cl.: A61L 27/40, A61L 27/58

(54) **Designed composite degradation for spinal implants**

(30) Priority: 18.12.2003 US 740055
(62) Divisional of application: 04812792.2
(71) Applicant: Warsaw Orthopedic, Inc., Warsaw, Indiana 46581 (US)
(72) Inventor: Moltz, Fred J. IV, Birmingham, AL 35242 (US); Sherman, Michael C., Memphis, TN 38120 (US)
(74) Representative: Hughes, Andrea Michelle

(57) **Abstract**

The invention includes a composite material for use in the construction of orthopedic devices and methods for using these composites. The composite material is comprised of at least one filament or cord that can be non-biodegradable and a biodegradable matrix. In other forms the composite material contains at least two components formed of a biodegradable material, which components can be a matrix or a filament or a combination of matrices and filaments. The degradation rate of the two components need not be the same. The composite material is used in the construction of orthopedic devices such as bone plates, bone rods, spinal rods, and laminate sheets that change physical properties in vivo.

## Description

### FIELD OF THE INVENTION

The present invention relates generally to composite materials to construct orthopedic devices for promoting bone fusion orthopedic devices.

The mammalian skeletal system, including long, short, flat, and irregular bones, is vulnerable to disease, injury, and congenital deficiencies, all of which can cause defects to the bone. Disease, injury, and deformity may have a disastrous impact on patient well being, ranging from acute pain to chronic debilitating pain.

Common treatments for defective bone tissue include joining or fusing fractured bone segments or portions together to stabilize the affected parts and can include removing and/or replacing portions of affected bone tissue, either in part or in whole. A bone plate or other prosthetic device can be inserted to eliminate disparate motion between the two bone portions to allow arthrodesis.

It is important, particularly for load-bearing bone, that the prosthetic device not stress shield the new bone growth and permit a weakened juncture or pseudoarthrodesis between the bone portions or adjacent vertebrae to be fused. It is known that for load bearing bone members, stronger, denser bone tissue results when new bone growth occurs under pressure. The problem arising is when and how to determine the amount of pressure or force desirable to develop a strong junction between the bone portions. The bone portions should be secured and supported during bone growth. However, the optimum support necessary for desired bone growth may vary over time as the bony juncture or bridge develops between the bone portions.

Similarly, stretched and/or torn ligaments can be treated by initially securing/immobilizing the ligaments. This can be accomplished using either, or both, internal and external prosthetic devices to augment or replace the stability lost as a result of the damage to the ligaments. Further, once-damaged ligaments can be susceptible to repeated injury. Consequently, it may be desirable to augment the treated ligament by implanting a prosthesis or device that allows limited movement of the affected spinal components while preventing the components from moving far enough to incur re-injury or cause new damage. Current treatment methods do not allow for an implanted device to initially secure or immobilize the ligaments and then allow limited movement of the same without a subsequent surgical revisitation.

In light of the above, there is a continuing need for materials for use in orthopedic devices, novel orthopedic devices, and treatments using these materials to stabilize and support damaged bone tissues, bony structures, and connecting tissue. There is also a need for materials, which provide variable loads to growing bone, as well as a measure of flexible support to injury or disease prone bones and connecting tissue. The present invention addresses these needs and provides other benefits and advantages in a novel and nonobvious manner.

### BRIEF SUMMARY OF THE INVENTION

The present invention relates to composite materials with anisotropic properties used to construct orthopedic devices, and the manufacture of these devices. Various aspects of the invention are novel, nonobvious, and provide various advantages. While the actual nature of the invention covered herein can only be determined with reference to the claims appended hereto, certain forms and features, which are characteristic of the preferred embodiments disclosed herein, are described briefly as follows.

In one form, the present invention provides an anisotropic composite material used to construct orthopedic devices. The composite material comprises: a bio-stable flexible cord configured to be fixedly secured to two or more bone portions allowing translational, or rotational, or both translational and rotational movement of a first one of the bone portions relative to a second one of the bone portions. A more rigid and more biodegradable material engages with the cord such that the biodegradable material restricts the translational, rotational, or both the translational and rotational movement of the first of the bone portions relative to the second of the bone portions secured to the composite material.

The composite material can be used to construct orthopedic devices used to treat a variety of bone defects including, but not limited to, bone fractures, diseased bone tissues, spinal diseases, diseased/damaged vertebrae, torn or stretched ligaments, and the like.

In preferred embodiments, the devices comprising the composite material prevent, or at least reduce, stress shielding of new, developing bone tissue. In other embodiments, the orthopedic device of the present invention can be configured for articulating joints. In these embodiments, the composite material can allow a limited amount of movement, i.e. translation and/or rotation about the joint. The devices, with and without the biodegradable material, still provide a measure of support and/or restriction of the movement of bone portions attached to devices comprising the composite materials. In preferred embodiments, the devices of the present invention remain in place indefinitely.

Further objects, features, aspects, forms, advantages, and benefits shall become apparent from the description.

### BRIEF DESCRIPTION OF THE DRAWINGS

FIG. 1 is a perspective view partly broken away of a composite material comprising an elongate cord including wound filaments and encased within a biodegradable matrix in accordance with the present invention.
FIG. 2 is a perspective view partly broken away of an alternative embodiment of an elongate composite material in accordance with the present invention
FIG. 3 is a perspective view of a plurality of non-biodegradable filaments supported by at least one biodegradable filament in accordance with the present invention.
FIG. 4 is a perspective view of a cord including a plurality of non-biodegradable filaments and at least one filament encased within a biodegradable matrix in accordance with the present invention.
FIG. 4A is a cross-sectional view of one of the filaments encased in a biodegradable matrix of the cord illustrated in Fig. 4.
FIG. 5 is a perspective view of a bone having a bone defect which has been treated using an orthopedic device prepared using one of the cords illustrated in FIGS. 1, 2, or 3.
FIG. 6 illustrates one embodiment of a composite material including a web material embedded within a biodegradable polymeric matrix.
FIG. 7 is a cross-sectional view of one embodiment of a composite material including a non-biodegradable cloth embedded between two biodegradable matrices in accordance with the present invention.
FIG. 8 is a cross-sectional view of an alternative embodiment of a fabric encased between two biodegradable matrices in accordance with the present invention.
FIG. 9 is a perspective view of a section of a spine, having a defect, which has been treated using a composite matrix in accordance with the present invention.

### DESCRIPTION OF THE PREFERRED EMBODIMENTS

For the purposes of promoting an understanding of the principles of the invention, reference will now be made to the embodiments illustrated herein, and specific language will be used to describe the same. It will nevertheless be understood that no limitation of the scope of the invention is thereby intended. Any alterations and further modifications in the described devices, systems, and treatment methods, and any further applications of the principles of the invention as described herein, are contemplated as would normally occur to one skilled in the art to which the invention relates.

In preferred embodiments, the present invention provides a composite material for use in the construction of an implantable orthopedic device or prosthesis used to facilitate support and repair of defective bone structures and/or connective tissue. The defective bone structures can be the result of damaged, traumatized, and/or diseased tissue. By use of the term "orthopedic device", it is intended to include within its meaning a device or implant that can be used to treat or repair defective, diseased, or damaged tissue of the muscular/skeletal system(s).

The biodegradable material of the present invention provides a composite material that includes a supporting matrix and a cord for an implantable orthopedic device. This supporting matrix can provide rigidity and support for both the implanted orthopedic fusion device and, consequently, the attached bone structures. In use, the biomechanical load supported by the composite material and/or orthopedic devices incorporating the composite can vary over time. This allows the orthopedic device to become dynamizable, or change its physical properties *in vivo.* This change in physical properties can be particularly important for developing strong, new bone tissue at the bone defection or fusion site. This prevents stress shielding of the new bone in-growth and minimizes the risk for the development of pseudoarthrodesis.

In one form, degradation of the matrix can occur naturally without the use of subsequent treatment. In other forms, degradation of the matrix can be initiated (or triggered), induced, and/or completed at a selected or predetermined time after implantation. The device and/or composite material can include a polymer susceptible to or sensitive to radiation energy, light (UV), solvents with different pH levels, thermal energy, or temperature, to initiate degradation. The treatment can include both invasive and non-invasive treatments. Preferably, the treatment can be accomplished using a UV radiation probe inserted in close proximity to the device (or composite material).

The following description specifically describes non-limiting, specific embodiments for use with the present invention.

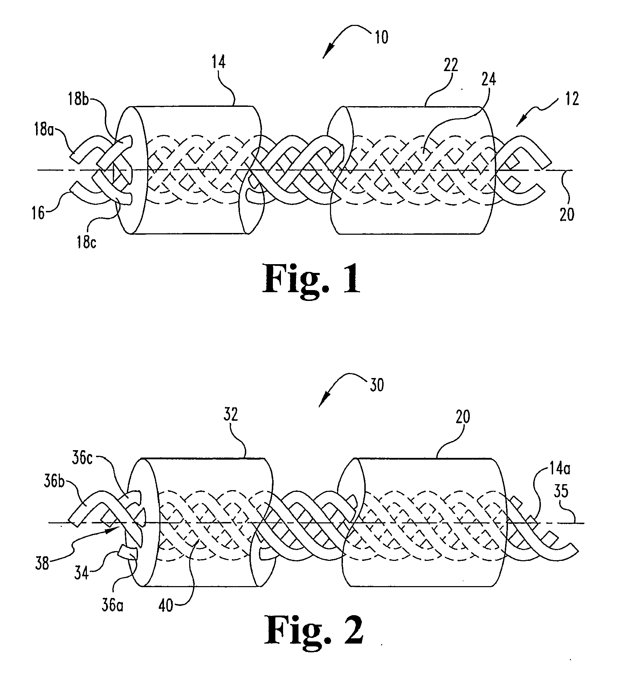

FIG. 1 is a perspective view of one embodiment of a composite material 10 including a cord 12 and a matrix 14. Cord 12 can be provided as a single elongate filament 16, or alternatively, as a plurality of filaments 18a, 18b, and 18c, ..., collectively referred to as filament 18. When cord 12 is a single filament, it can be provided as a large diameter rod or solid core encased within matrix 14. Implant 10 defines a longitudinal axis 20. In preferred embodiments, cord 16 and/or individual filaments 18a, 18b, 18c, extend substantially in the direction of longitudinal axis 20. Although it will be understood that one or more of individual elements 18a, 18b, 18c, ..., while extending generally in the direction of longitudinal axis 20, can either wind around that direction and extend substantially orthogonal or at an angle oblique to that direction at any given location within implant 10. In other embodiments, the plurality of filaments 18a; 18b, 18c, ... can be woven together to provide a flat mesh or a three-dimensional network of filaments.

Matrix 14 can substantially encase cord 12. Alternatively, at least a portion of cord 12 can extend through or beyond the surface of matrix 14. Matrix 14 can provide support to maintain a desired shape for an orthopedic device. Consequently, matrix 14 can be provided as a variety of biodegradable materials. Some of the materials can be readily formable in the operating room, for example, by heating the material and shaping the composite into a desired configuration to either conform to the bone defect and/or to induce the bone defect to be retained in a desired configuration. Alternatively, matrix 14 can be pre-formed or shaped by the supplier or manufacturer. Matrix 14 is illustrated as a substantially cylindrical elongate configuration. It should be understood that matrix 14 can be provided in any desirable configuration including as a substantially bent, planar, or flat configuration. Alternatively, matrix 14 can be provided in any desirable shape including a substantially spherical, square, rectangular, or amorphous configuration, which, as noted above, may or may not be moldable by hand either at elevated temperatures or under other conditions including light, moisture, or solvent activated.

In alternative embodiments, matrix 14 is bonded to cord 12. A biocompatible chemical adhesive can be used to bond the matrix and cord 12 together. The bond can also be derived from a mechanical interlock between the matrix 14 and the cord 12.

While composite 10 is illustrated as an elongate cylinder, it will be understood that other configuration are contemplated and are intended to be included within the scope of the present invention. For example composite 10 can be bent, planar, cuboid, spherical or of an amorphous shape as desired. Further composite 10 (and cord 12) can include various structures to permit it to be secured to bone tissues. Examples of various structures include without limitation: eyelets, loops, hooks, bone fasteners, pins, pegs, cements, glues, and combinations thereof

Cord 12 extends through at least a portion of matrix 14. Cord 12 can be formed or composed of a variety of individual filaments either separated from each other in matrix 14 or in direct contact with each other or loosely bundled together. Filaments 18a, 18b, 18c, ... can be braided or woven together and extend at least partially through matrix 14. Alternatively, filaments 18a, 18b, 18c, ... can extend parallel to each other through at least a portion of matrix 14. In still other embodiments, cord 12 and/or filament 18 can be substantially embedded within and completely surrounded by matrix 14, such that no portion of the cords or filaments are exposed or visible.

Each of filaments 18a, 18b, 18c, ... can be formed of the same material and/or of the same shape, diameter, and length. Alternatively, one or more of 18a, 18b, 18c, ... can be provided as a different material or formed in a different shape, diameter, length, or configuration as desired. Providing the individual filaments 18a, 18b, 18c, ... in different materials, shapes, and sizes can induce the implant to produce different desirable physical properties and, consequently, an orthopedic implant can be prepared tailored to treat the individual orthopedic defect or disease.

In one embodiment, cord 12 is elastic and/or flexible. Consequently, one or more of filaments 18a, 18b, 18c, ... can be an elastic or flexible material. Weaving the filaments 18a, 18b, 18c, ... together can modify the cord's elasticity or flexibility. For example, using either a loose weave or a tight weave, differing sizes of spaces 24 can exist between the individual filaments 18a, 18b, 18c, ... and can allow cord 12 to exhibit varying degrees of flexibility.

Cord 12 (and filaments, 18a, 18b, 18c ...) can exhibit a smooth exterior surface. Alternatively, cord 12 (and filaments, 18a, 18b, 18c ...) can exhibit an exterior surface that is roughened pitted, grooved, or knurled. The textured exterior surface of cord 12 can facilitate bonding the matrix material to the cord via a mechanical interlocking mechanism either solely or in conjunction with an adhesive. The three dimensional network of the filaments 18a, 18b, 18c ... making up cord 12 can include voids or spaces which can also facilitate bonding the matrix material 14 to the cord 12 via a mechanical interlocking mechanism. Additionally the surface of either matrix 14 or the cord 12 can be treated to facilitate good adherence. Such surface treatment can include corona discharge, plasma discharge, chemical etching, electron or ion beam radiation, and laser radiation, and the like as is known in the art.

Cord 12 can be provided as a non-biodegradable material. Examples of non-biodegradable materials are discussed more fully below. In addition, cord 12 can include one or more individual filaments, which may be composed of a biodegradable material. The biodegradable material for the filaments can compose a shape memory polymer, and/or other biocompatible polymeric material.

In one preferred embodiment, matrix 14 is composed of a biodegradable material 22. *In vivo,* matrix 14 erodes or biodegrades. As matrix 14 biodegrades, the rigidity of composite 10 decreases. In preferred embodiments, this decrease in rigidity is substantially linear over time. As discussed more fully below, the nature and composition of matrix 14 can be varied to allow matrix 14 to degrade over varying time periods including periods between a few days, a few weeks, a few months, and even over the course of one or more years. Matrix 14 can be formulated to have a desired half-life *in vivo*. By use of the term "half life", it is intended to mean that matrix 14 degrades to about one-half of its initial mass in the specified time period. In one preferred embodiment, matrix 14 has a half-life, *in vivo,* of less than about 6 months; more preferably, matrix 14 has a half-life of less than about 12 months; still more preferably, matrix 14 has a half-life of less than about 18 months. In other embodiments, matrix 14 can be formulated to have a half-life that is greater than or equal to one year; more preferably greater than or equal to 18 months.

FIG. 2 is a perspective view of an alternative embodiment of an elongate composite material 30 in accordance with the present invention. Elongate composite 30 defines a central axis 35. Composite material 30 includes matrix 32 and a cord 34 engaged therein and extending generally along the axis 35. Cord 32 can comprise a single filament 36a or a plurality of filaments 36a, 36b, 36c, ..., collectively referred to as filament 36. In the illustrated embodiment, filaments 36a, 36b, 36c, ... are wound together to provide cord 34.

Generally, composite material 30 can be provided substantially as has been described above for composite material 10, including the description of the matrix 22 and and/or filaments 18a, 18b, 18c, ... The winding of filaments 36a, 36b, 36c,... can provide differing properties of that exhibited by the braiding of filaments 18a, 18b, and 18c including the ability to define a central cavity 38 therein. Central cavity 38 extends substantially parallel to axis 35. In one embodiment, central cavity 38 is substantially filled with the material of matrix 34. In other embodiments, yet another filament or cord can extend through central cavity 38. In effect, filaments 36a, 36b, 36c, ... can be wound around the central cord or filament. The central cord or filament can be the same or different from either cord 34 or filament 36. Additionally, the winding of filaments 36a, 36b, 36c, ... also generates additional spaces or voids 40 between individual filaments, for example, between filaments 36a and 36b. In still other embodiments cavity 38 can be filed with a therapeutic agent or osteogenic material.

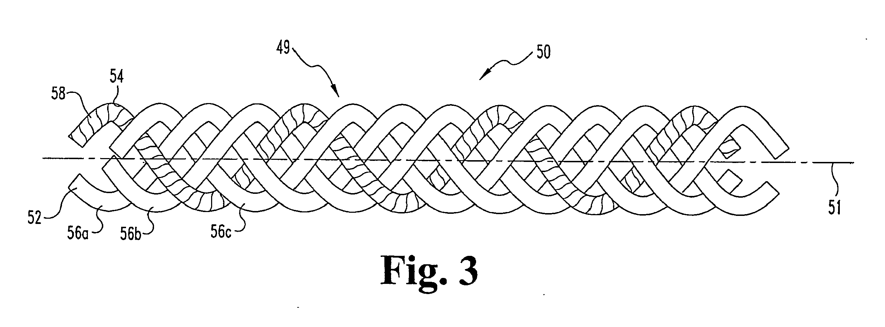

FIG. 3 is a perspective view of one embodiment of a composite material 49 that includes a tether or cord 50 in accordance with the present invention. Cord 50 comprises a plurality of filaments extending generally along a central axis 51. In preferred embodiments, cord 50 includes a first set of filaments 52 and at least a second set of filaments 54. Other sets or individual filaments can also be included within cord 50. In the illustrated embodiment, first set of filaments 52 can include a plurality of individual filaments 56a, 56b, 56c ... Filaments 56a, 56b, 56c ... can be the same filaments and can have the same length or configuration. Alternatively, a select one or more of filaments 56a, 56b, 56c ... can be different from the other filaments in either composition, physical properties, size, diameter, length, and the like. First set of filaments 52 can be provided substantially as has been described for filament 18 (and for cord 12). Additionally, it will be understood that the relative arrangements of filaments 56a, 56b, 56c ... can be either provided as a plurality of parallel filaments, wound filaments, braided filaments, and the like. One or more of filaments 56a, 56b, 56c ... can be provided as a substantially rigid filament formed of a non-biodegradable material, which is discussed in more detail below.

Cord 50 also includes a second set of filaments 54. Second set of filaments 54 can include a single filament 58 or a plurality of filaments arranged similarly to that discussed above for first set of filaments 52.

Filament 58 can be composed of a biodegradable material, discussed more fully below. Additionally, filament 58 can be a substantially rigid filament that provides support for cord 50 and/or lends further support to individual filaments of the first set of filaments 52. In the illustrated embodiment, filament 58 is provided to substantially interweave or woven into the plurality of filaments 56a, 56b, 56c ... In other embodiments, filament 58 can be provided to extend substantially parallel to one or more filaments of the first set of filaments 52, wrap around one or more filaments of the first set of filaments 52, and/or be spirally wound within the first set of filaments 52. Filament 58 can be provided to degrade *in vivo* at a desired degradation rate or within a desired time period. The degradation rate or the half-life of filament 58 can be tailored to suit the particular need, treatment, and/or application of cord 50. In one embodiment, the half-life of filament 58 is selected to be greater than about 6 months; more preferably, greater than or equal to about 1 year; still yet more preferably, greater than or equal to about 18 months. In other embodiments, filament 58 can be provided to have a half-life of less than about 1 year. Furthermore, filament 58 can be provided to have substantially the same configuration, length, diameter, mass, and/or tensile strength as that exhibited by either the individual filaments of the first set of filaments 52 and/or one ore more filaments 56a, 56b, 56c ...

In use, as the filaments of the second set 54 degrade *in vivo,* the rigidity of cord 50 and/or one or more of the individual filaments of the first set 52 can be decreased. This allows cord 50 and/or one or more filaments of the first set 52 to become more flexible. Consequently, if the bone portions to which cord 50 and/or the first set of filaments 52 are attached articulate, the flexibility or increasing flexibility over time allows increased movement of the articulating joint as new bone tissue grows and the defect is corrected. It will be understood that in preferred embodiments cord 50 remains secured to the bone portions albeit minus some or all of the filaments of the second set 54. Furthermore, it will be understood that in other aspects, cord 50 can be substantially as provided as described above for cords 12 and 34.

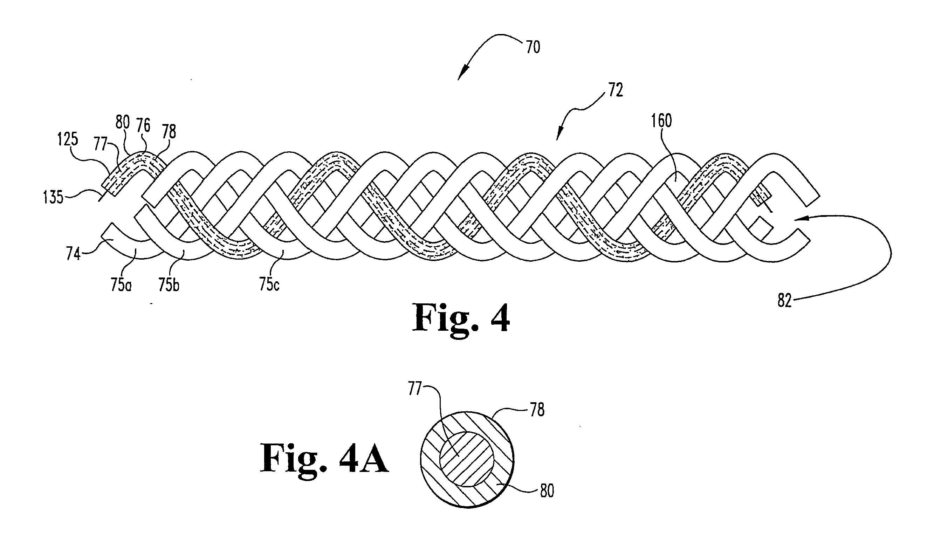

FIG. 4 is a perspective view of an alternative embodiment of a composite material 70 for use in forming orthopedic devices in accordance with the present invention. Composite material 70 includes a cord 72 comprising a first set of filaments 74 and at least a second set of filaments 76. First set of filaments 74 can be provided substantially as has been described above for first set of filaments 52 for cord 50 and can include a plurality of individual filaments 75a, 75b, 75c, ... Second set of filaments 76 can comprise one, two, three, or more filaments, collectively referred to as filament 78. Referring additionally to Fig. 4A, filament 78 includes at least an outer coating or matrix 80 composed of a biodegradable material, discussed more fully below and an inner core material 77 that comprises one of: a large diameter rod, a solid core, a smaller wire, filament, braid, or plurality of filaments as desired. In one embodiment, the inner core material 77 can comprise a filament of cord similar to that defined by the first set of filaments 74. Alternatively, inner core material 77 can be the same or can be different from any one of filaments 75a, 75b, 75c, ... Additionally, core 77 can either be formed of a biodegradable material and/or a non-biodegradable material, both of which are discussed more fully below. Filament 78 including core material 77 and matrix 80 can be substantially rigid or provide rigidity to cord 72.

As matrix 80, comprising a biodegradable material, begins to erode, *in vivo,* the rigidity of filament 78 and/or core 77 begins to decrease. Consequently, the rigidity of cord 72 also begins to decrease. This allows the bone portions to which an implant is attached to articulate or carry an increasing amount of load to promote formation of hard cortical bone tissue and prevent pseudoarthrodesis. In other aspects, such as rigidity, size, configuration, diameter, half life, and the like, filament 78 can be provided substantially as has been described above for any one of the filaments 58 or cord 50. Additionally, cord 72 can be encased or substantially encased within a matrix such as matrix 14 or 32 of composite material 10 or 30, respectively.

One or more of filaments 75a, 75b, 75c and filament 78 can be bundled together to define an interior region 82 therein. Interior region can be a void, contain the matrix material, or a therapeutic agent, osteogenic material or another cord of plurality of filaments as discussed above for cavity 38. In other embodiments, the plurality of filaments 75a, 75b, 75c, ... can be woven together to provide a flat mesh or three-dimensional network of filaments.

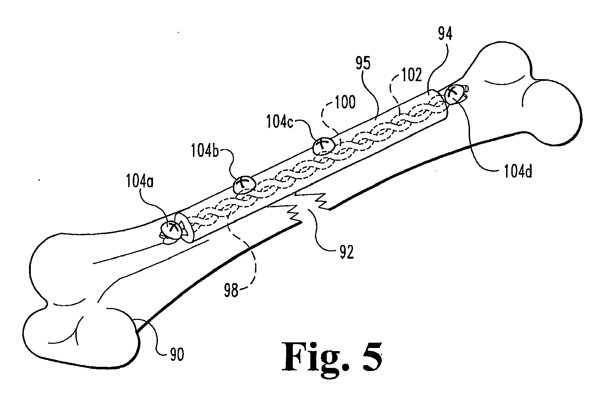

FIG. 5 is a perspective view of one embodiment of a bone 90 having a defect 92 therein. An orthopedic implant 94 comprised of an elongate composite material 95 is illustrated as attached to bone 90 and spanning defect 92. Orthopedic implant 94 can be comprised of a composite material as has been discussed above such as any one of composite materials 10, 30, 70 or cords 50 or 72 described above. In the illustrated embodiment, orthopedic implant 94 includes an outer matrix 96 substantially encasing a cord 98. Cord 98 comprises a first filament 100 and a second filament 102. The orthopedic implant 94 can be attached to the bone portions by any means commonly used and/or known in the art including, without limitation, bone screws 104a, 104b, 104c, and 104d, staples, wire, cable, and the like. It will be observed from the illustration that some of screws, such as 104a and 104d, can extend solely through cord 98 with or without going through matrix 96. Other screws, such as those listed as 104b and 104c, may extend through outer matrix 96 and may or may not contact cord 98. In use, outer matrix 96 slowly degrades, *in vivo.* After degrading, the residual portion of the implant, i.e., cord 98, can remain secured to the bone portions to provide additional support and/or restraint. However, as noted above and discussed more fully below, degradation of outer matrix 96 can allow increasingly greater stress on new bone growth within defect 92. This can provide optimal bone tissue growing conditions to ensure hard, dense cortical bone grows into the defect. In addition, an osteogenic material can be added to the bone defect, either supplied separately, combined with the outer matrix, and/or incorporated into the cord.

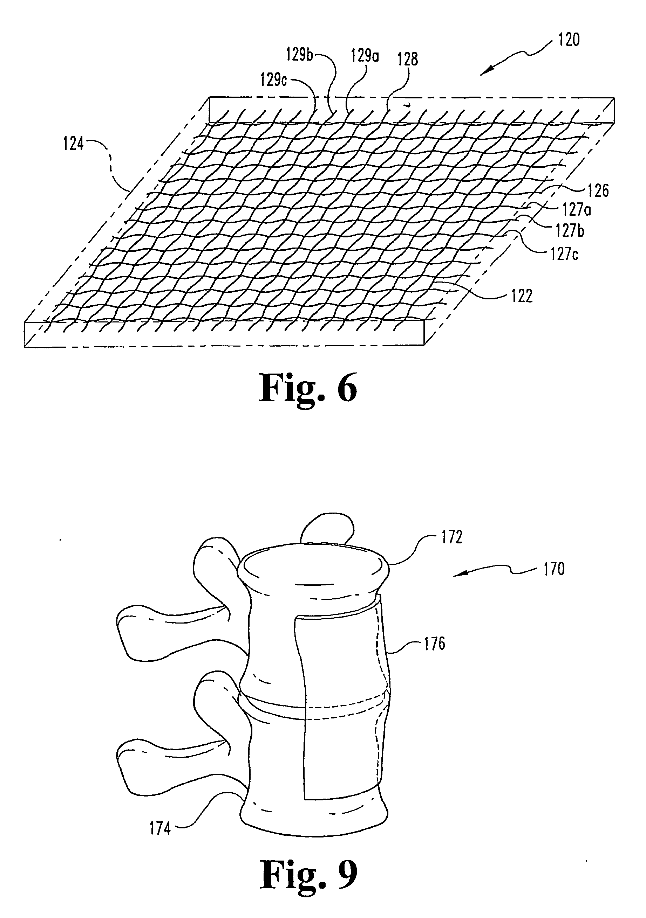

FIG. 6 is perspective view of another embodiment of composite material 120 for use in the present invention. Composite material 120 comprises a woven or an array of cords to provide a mesh 122 and a matrix 124. Mesh 122 can be a flat (two-dimensional), fabric, or cloth-like material or three-dimensional network. Matrix 124 can be formed similarly as described above for matrices 96, 80, and 14. Consequently, matrix 124 can be a biodegradable or bioerodable material that can provide rigid support to the orthopedic implant formed from the composite material 120.

The mesh 122 can comprise a first set of filaments 126 and at least a second set of filaments 128. In the illustrated embodiment, first and second sets of filaments 126 and 128 are provided to lie substantially orthogonal to each other. It will be understood by those skilled in the art that the relative orientation of first set of filaments 126 and second set of filaments 128 can be provided as desired, including substantially parallel to each other, woven, braided, or oriented at an angle oblique to each other. Furthermore, first set of filaments 126 and second set of filaments 128 can comprise substantially the same material or comprise a different material from each other. Furthermore, first set of filaments 126 and second set of filaments 128 can have substantially the same properties including tensile strength, diameter, length, shape, and the like, or the two sets of filaments can have different tensile strength, diameter, length, shape and the like from each other. Additionally, first set of filaments 126 can be provided substantially as described above for first set of filaments 74 and/or first set of filaments 52. Similarly, second set of filaments 128 can be provided substantially as has been described above for first set of filaments 74 and 52, or second set of filaments 76 and/or 54.

First set of filaments 126 and second set of filaments 128 can be engaged with or secured to each other. The engagement can be in the form of bonding with or without glue, woven together, knotted together, overmolded on top of each other, or secured via a mechanical interlocking mechanism as desired.

In the illustrated embodiment, first and second sets of filaments 126 and 128, respectively, are substantially encased within matrix 124. It will be understood that one or more, or both, of first set of filaments 126 and second set of filaments 128 can be exposed or at least partially exposed extending out of matrix 124.

First set of filaments 126 can comprise a plurality of filaments 127a, 127b, 127c, ... and each filament can be composed of the same material and/or exhibit the same physical properties, size, and shape. Alternatively, each of filaments 127a, 127b, 127c, ... can be of a different material or of a different size, shape, or physical properties as desired.

Similarly, the individual filaments 129a, 129b, 129c, ... making up of the second set of filaments 128 can be of the same materials and/or same physical properties and sizes or they can be of different materials, sizes, and/or physical properties as desired.

In another embodiment, the first set of filaments 126 and second set of filaments 128 are composed of different materials and/or having different physical properties, sizes, and shapes. This can be used to prepare an orthopedic matrix having anisotropic properties, i.e., exhibiting different properties in different directions. For example, the second set of filaments 128 can comprise a biodegradable or non-biodegradable material. For example, the first and second set of filaments 126 and 128 can both be composed of biodegradable material either the same or different second material. The degradation rates or half-lives of the two materials may be different.

Alternatively, the first set of filaments 126 can be composed of a biodegradable material while the second set of materials are composed of a non-biodegradable material. Consequently, the second set of filaments 128 remains *in vivo* while the first set of filaments 126 erode away.

In yet another embodiment, the size and/or shape of the filaments in the first set of filaments 126 can be different from the filaments in the second set of filaments 128. One set of filaments can persist *in vivo* for a longer period of time.

This provides an orthopedic implant having various properties, which properties can be tailored to suit the particular application and treatment method used on the orthopedic defect.

Matrix 124 can be provided as a moldable or shapeable material that can be rigid *in vivo* and at ambient temperature and/or under pharmacological conditions. However, if desired, matrix 124 can be formulated to be hand or machine moldable either at an elevated temperature within a specified solvent or under specific conditions. For example, the matrix material 124 can comprise one or more cross-linkable polymeric materials such that upon initiation, the matrix material forms a cross-linked matrix having the desired or preformed configuration. Matrix material 124 can be bonded or secured to first set of filaments 126 and/or the second set of filaments 128 as desired with or without glue, overmolded, or secured via a mechanical interlocking mechanism.

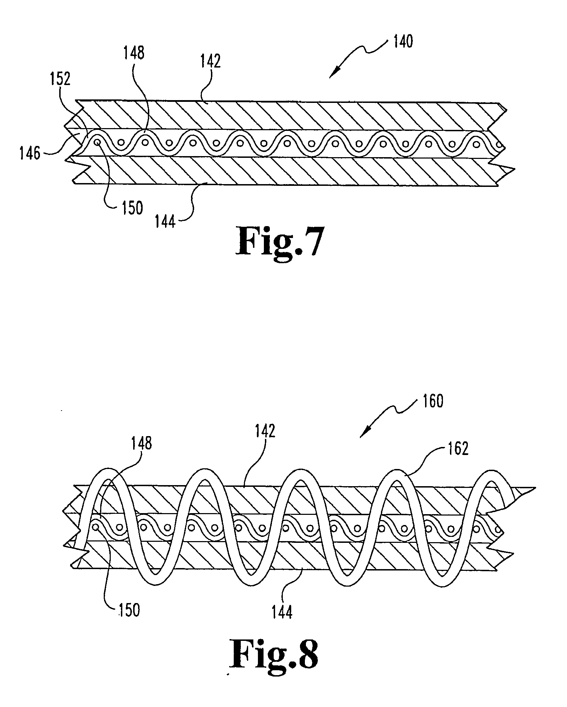

FIG. 7 is a cross-sectional view of one embodiment of a composite material 140 for use in the present invention. Composite material 140 can be provided substantially as has been described above for composite material 120. Alternatively, composite material 140 can be different from that described above. For example, composite material 140 can comprise a first matrix 142 and at least a second matrix 144. First and/or second matrix can be made of the same or different material. For example, first matrix 142 can be formed of a first biodegradable material, and second matrix 144 can be formed of a second biodegradable material. Additionally, a loose weave or cloth-like material 146 comprising a first set of filaments, 150 and a second set of filaments 152. The weave or cloth-like material 146 can be disposed in between first matrix 142 and at least a second matrix 144. Weave or material 146 can be provided substantially as has been described above for mesh 122. In selected embodiments, one or both of first matrix 142 and second matrix 144 can be bonded, over molded, or engaged to woven material 146, and more specifically, to fibers 148 composing the woven material 146. In other embodiments, first matrix and/or second matrix can be glued using a biocompatible adhesive to one or more of the woven material 146 and/or fibers 148.

Referring additionally, to FIG. 8, a composite material 160 is illustrated. Composite material 160 is similar to that illustrated above for composite material 140. Consequently, like reference numbers will be used to denote like components. Composite material 160 comprises a first matrix 142 and a second matrix 144 and a woven material 146 between. Additionally, a third set of filaments 162 is illustrated as a weaving or suturing to bind together first matrix 142, second matrix 144, and woven material 146. Third set of filaments 162 can be comprised substantially as has been described above for the first set of filaments 74, 52, 36, and 18. Alternatively, third set of filaments 162 can be provided as has been described above for second set of filaments 77 and 58. In yet another alternative, first matrix 142, second matrix 144, and woven material 146 can be fastened together by any means commonly used or known in the art including cords, strings, filaments, staples, clips, ties, bands, glues, cements, and combinations thereof.

FIG. 9 is an illustration of a portion of a spinal column 170 with a defect and including a first vertebrae 172 and a second vertebrae 174. The bone defect can be treated using an orthopedic device 176. Orthopedic device 176 can comprise a material such as that described above for composite material 160, 140, and/or 120. In use, as the biodegradable component of orthopedic device 176 degrades, the residual component, i.e., either a woven matrix and/or a portion of a woven matrix, can remain secured to one or both of first and second vertebrae 172 and 174, respectively. This can allow the two vertebrate to articulate relative to each other, yet maintain the integrity and restrict movement or allow limited movement of the spinal column.

The biodegradable material included in one or more cords, filaments, and/or matrices described above can be formed or composed of a variety of materials including, without limitation, degradable or resorbable polymeric materials, composite materials, and ceramic materials.

In one embodiment, the biodegradable material can include polymeric materials formed from oligomers, homopolymers, copolymers, and polymer blends that include polymerized monomers derived from 1, d, or d/l lactide (lactic acid); glycolide (glycolic acid); ethers; amino acids; anhydrides; orthoesters; hydroxy esters; and mixtures of these monomeric repeating units.

Use of the term "copolymers" is intended to include within the scope of the invention polymers formed of two or more unique monomeric repeating units. Such copolymers can include random copolymers; graft copolymers; body copolymers; radial body, dibody, and tribody copolymers; alternating copolymers; and periodic copolymers. Use of the term "polymer blend" is intended to include polymer alloys, semi-interpenetrating polymer networks (SIPN), and interpenetrating polymer networks (IPN).

In a preferred embodiment, the biodegradable material comprises a biodegradable polymeric material including: poly(amino acids), polyanhydrides, polycaprolactones, poly(lactic-glycolic acid), polyhydroxybutyrates, polyorthoesters, and poly(d,l-lactide).

In other embodiments, the biodegradable material can comprise biodegradable ceramic materials and ceramic cements. Examples of biodegradable ceramic materials include: hydroxyapatite, hydroxyapatite carbonate, corraline, calcium phosphate, tricalcium phosphatem, and hydroxy-apatate particles. Examples of biodegradable ceramic cements include calcium phosphate cement. Such calcium phosphate cements are preferably synthetic calcium phosphate materials that include a poorly or low crystalline calcium phosphate, such as a low or poorly crystalline apatite, including hydroxyapatite, available from Etex Corporation and as described, for example, in U.S. Patent Nos. 5,783,217; 5,676,976; 5,683,461; and 5,650,176, and PCT International Publication Nos. WO 98/16268, WO 96/39202 and WO 98/16209, all to Lee et al. Use of the term "poorly or low crystalline" is meant to include a material that is amorphous, having little or no long range order, and/or a material that is nanocrystalline, exhibiting crystalline domains on the order of nanometers or Angstroms.

In still other embodiments, the biodegradable material can be formed of composite materials. Examples of composite materials include as a base material or matrix, without limitation: ceramics, resorbable cements, and/or biodegradable polymers listed above. Each of the base materials can be impregnated or interspersed with fibers, platelets, and particulate reinforcing materials.

In one form, the biodegradable material comprises a resorbable, moldable material that can be molded at an elevated temperature and then allowed to set up into a hardened material at around body temperature, such as the material sold under the trade name BIOGLASS® discussed in WO 98/40133.

The composite material of the present invention can be tailored to degrade at a predetermined or pre-selected rate by suitably selecting the size, thickness, and/or biodegradable material. In preferred embodiments, the biodegradable material degrades at a rate comparable to the new bone in-growth into the bone defect or bone fusion site. In particularly preferred embodiments, the rigid biodegradable component has an *in vivo* half life of greater than three months, more preferably the *in vivo* half life of the restricting component is greater than six months; still more preferably the *in vivo* half life is greater than one year. By use of the term "half life", it is understood that the degradation rate of the restricting component is such that the restricting component loses half of its initial mass *in vivo,* presumably due to resorption, degradation, and/or elimination.

Further, the biodegradable material can be formulated to degrade or can be induced to begin degradation by application of external stimuli. For example, the biodegradable material can degrade upon application of radiation such as UV radiation, thermal energy, and/or solvents
--either neutral, basic, or acidic.

A nonbiodegradable or biostable material for use in the present invention can include resilient materials such as, without limitation, nitinol, titanium, titanium-vanadium-aluminum alloy, cobalt-chromium alloy, cobalt-chromium-molybdenum alloy, cobalt-nickel-chromium-molybdenum alloy, biocompatible stainless steel, tantalum, niobium, hafnium, tungsten, and alloys thereof; polymeric materials include polymerized monomers derived from: olefins, such as ethylene, propylene, butene-1, pentene-1, hexene-1, 4-methylpentene-1, styrene, norbornene and the like; butadiene; polyfunctional monomers such as acrylate, methacrylate, methyl methacrylate; esters, for example, caprolactone and hydroxy esters; and mixtures of these monomeric repeating units. Preferred polymers for use in the present invention include carbon poly(ether, ether, ketone) (PEEK), poly(aryl ether, ketone) (PAEK), and the like.

In addition or in the alternative, it may be desirable to promote bone fusion between the adjacent vertebrae or between any bone portions on either side of a bone defect. In this embodiment, it may be desirable to include an osteogenic material or a bone growth material such as an osteoinductive or an osteoconductive material. For example, it may be desirable to introduce an osteogenic factor such as a bone morphogenic protein (BMP) or a gene encoding the same operationally associated with a promoter which drives expression of the gene in the animal recipient to produce an effective amount of the protein. The bone morphogenic protein (BMP) in accordance with this invention is any BMP able to stimulate differentiation and function of osteoblasts and osteoclasts. Examples of such BMPs are BMP-2, BMP-4, and BMP-7, more preferably rhBMP-2 or rhBMP-7, LIM mineralization protein (LMP) or a suitable vector incorporating a gene encoding the same operably associated with a promoter, as described in WO99/06563 (see also Genbank accession No. AF095585).

The composite materials and orthopedic devices of the present invention can be used by themselves or in conjunction with one or more known orthopedic devices as deemed medically prudent. Additionally or in the alternative, the present invention can be used with one or more devices disclosed in co-pending US patent Publication 2005-0085814 filed on October 21, 2003 entitled, "Dynamizable Orthopedic Implants and Their Use in Treating Bone Defects."

In preferred embodiment, the composite material of the present invention can provide initial support and/or fixation of selected bone structures. After a selected period of time or under certain conditions, the amount and nature of the support/fixation can vary to facilitate a desirable treatment. For example, use of a composite material according to the present invention allows that variable or dynamizable support develops new, strong bone tissue, thus minimizing the risk of pseudoarthrodesis.

The composite material of the present invention also finds advantageous use in the treatment of connecting tissue such as ligaments. For example, devices comprising the composite material can augment connecting tissue. After a predetermined period of time or condition, the composite material can allow limited translational, rotational, or translational and rotational movement of the connecting tissue and/or bone structures attached to the orthopedic device incorporating the composite. For example, if the natural connecting tissue is elastic, the composite material can serve to limit or restrict the overall length or amount that the connecting tissue stretches. This restriction can vary depending upon the length of time or pre-selected conditions used in forming the composite material used in constructing and using the device.

While the invention has been illustrated and described in detail in the drawings and foregoing description, the same is considered to be illustrative and not restrictive in character, it is understood that only the preferred embodiments have been shown and described.

Any reference to a specific directions, for example, references to up, upper, down, lower, and the like, is to be understood for illustrative purposes only or to better identify or distinguish various components from one another. These references are not to be construed as limiting in any manner to the orthopedic device and/or methods for using the orthopedic device as described herein.

Unless specifically identified to the contrary, all terms used herein are used to include their normal and customary terminology. Further, while various embodiments of medical devices having specific components and structures are described and illustrated herein, it is to be understood that any selected embodiment can include one or more of the specific components and/or structures described for another embodiment where possible.

Further, any theory of operation, proof, or finding stated herein is meant to further enhance understanding of the present invention and is not intended to make the scope of the present invention dependent upon such theory, proof, or finding.

## Claims

1. A composite material for use in orthopedic devices, said composite material comprising:
a flexible cord comprising a non-biodegradable material; and
a rigid matrix comprising a biodegradable material wherein said rigid matrix is in contact with said flexible cord such that said rigid matrix restricts the flexibility of said cord.

2. The composite material according to claim 1, wherein said matrix encapsulates said cord.

3. The composite material according to claim 1, wherein said matrix is bonded to said cord.

4. The composite material according to claim 1 wherein the cord exhibits an exterior surface that is roughened, pitted, grooved or knurled.

5. The composite material according to claim 4 wherein the matrix is secured to the cord via a mechanical interlock.

6. The composite material according to claim 1 wherein the cord has an exterior surface that has been treated to promote adhesion to the matrix.

7. The composite material according to claim 1, wherein said cord extends through said matrix.

8. The composite material according to claim 1, wherein said cord extends from the interior of said matrix to the exterior of said matrix.

9. The composite material according to claim 1, wherein said cord consists of a single elongate filament.

10. The composite material according to claim 1, wherein said cord comprises a plurality of elongate filaments braided together.

11. The composite material according to claim 1, wherein said cord comprises a plurality of elongate filaments wound together.

12. The composite material according to claim 1 wherein said cord comprises a plurality of elongate filaments woven together to provide a flat mesh or a three dimensional network of filaments.

13. The composite material according to claim 1, wherein said non-biodegradable material comprises material selected from the group consisting of:
poly(ether, ether, ketone), poly(aryl ether), poly(aryl ether ketone) polyethers, polyanhydrides, polyolefins, polyacylate, polymethacrylate, polymethylmethacrylate, polyesters, and copolymers and blends thereof.

14. The composite material according to claim 1, wherein said biodegradable material comprises material selected from the group consisting of: poly(amino acids), polyanhydrides, polycaprolactones, polylactates, polyglycolates, poly(lactic-glycolic acid), polyorthoesters, hydroxyapatite carbonate, corraline, calcium phosphate, tricalcium phosphatem, and blends thereof.

15. The composite material according to claim 1, wherein said biodegradable material is provided to have an initial mass and *in vivo* biodegrades to less than or equal to about half its initial mass within about one year.

16. The composite material according to claim 1, wherein said biodegradable material is provided to have an initial mass and *in vivo* retains more than about half its initial mass for more than one year.

17. The composite material of claim 1, further including:
a bone growth material wherein said rigid matrix is impregnated with said bone growth material to promote arthrodesis.

18. The composite material of claim 1, further including:
a bone growth material wherein said cord is impregnated with said bone growth material to promote arthrodesis.

19. The composite material of claim 1, wherein said cord further includes a non-biodegradable structure for fixedly securing said orthopedic devices incorporating said composite material to portions of bone, wherein said structure is selected from the group of structures including, eyelets, loops, hooks, bone fasteners, pins, pegs, cements, glues, and combinations thereof.

20. The composite material of claim 1, wherein said orthopedic device is attached to bone portions and at least some of the load on said orthopedic device is transferred to bone portions as said rigid matrix biodegrades.

21. The composite material of claim 1, wherein said orthopedic devices are attached to bone portions, and said orthopedic devices allow restricted translational, or rotational, or translational and rotational movement of said bone portions after said rigid matrix biodegrades.

22. The composite material according to claim 1, wherein said cord comprises a plurality of elongate filaments aligned substantially parallel to each other.

23. The composite material according to claim 22, wherein said elongate filaments are secured together.

24. A method of making orthopedic devices to secure to bone portions, said method comprising:
providing the composite material according to claim 1; and
incorporating said composite material into said orthopedic devices such said that the composite material restricts the motion of bone portions attached to said orthopedic devices.

25. The method of claim 24, wherein said orthopedic device is configured in the shape of a bone plate.

26. The method of claim 24, wherein said orthopedic device is configured in the shape of a rod.

27. The method of claim 24, wherein said composite material is incorporated into said orthopedic devices such that said composite materials restrict the translational or rotational, or translation and rotational movement of the bone portions attached to said orthopedic devices before and after said rigid matrix biodegrades.

28. A method of treating a bone defect, said method comprising:
constructing an orthopedic device comprising the composite material of claim 1,
attaching said orthopedic device to two or more bone portions such that said rigid matrix in said composite material restricts the motion of two or more bone portions attached to said orthopedic device.

29. A composite material for use in orthopedic devices, said composite material comprising:
a woven material including a first set of elongate filaments and a second set of elongate filament each filament is comprised of a non-biodegradable material; and
a rigid matrix comprising a biodegradable material, wherein said woven material is over-laid with said rigid matrix such that said matrix restricts the movement of said first set of elongate filaments relative to said second set of elongate filaments.

30. A composite material comprising:
a plurality of flexible elongate filaments each comprised of a non-biodegradable flexible material; and
a plurality of rigid elongate filaments each comprised of a biodegradable material, wherein said flexible elongate filaments and said rigid elongate filaments are engaged with each other such that said rigid elongate filaments restrict the movement of said flexible elongate filaments.

31. The composite material according to claim 30, wherein said flexible elongate filaments and said rigid elongate filaments are braided together.

32. The composite material according to claim 30, wherein said flexible elongate filaments and said rigid elongate filaments are wound together.

33. The composite material according to claim 30, wherein said flexible elongate filaments and said rigid elongate filaments are aligned substantially parallel to each other.

34. The composite material according to claim 30, wherein said flexible elongate filaments and said rigid elongate filaments are woven together to provide a flat mesh or a three dimensional network of flexible and rigid filaments.

35. The composite material according to claim 30, further including:
a connecting component, wherein said flexible elongate filaments and said rigid elongate filaments are connected together by said connecting component, said connecting component selected from the group of connecting components consisting of cords, strings, filaments, staples, clips, ties, bands, glues, cements, and combinations thereof.

36. The composite material according to claim 30 wherein the flexible elongate filaments and the rigid elongate filaments are connected together with a biocompatible adhesive.

37. The composite material according to claim 35, wherein said connecting component is biodegradable.

38. A composite material, comprising:
a first set of flexible cords comprised of a biodegradable material;
a second set of flexible cords comprised of a biodegradable material; and
a rigid matrix that is more readily biodegradable than said flexible cords, wherein said first set of flexible cords are woven together, said second set of flexible cords are woven together, and said first set of flexible cords and said second set of flexible cords are attached to said rigid matrix.

39. A composite material comprising:
a first set of elongate filaments comprised of a rigid material;
a second set of elongate filaments comprised of a flexible material; and
a rigid matrix composed of a biodegradable material that exhibits a first degradation rate *in vivo*, wherein said first elongate filaments and said second elongate filaments are woven together to form a mesh and said rigid matrix is engaged with said mesh.

40. The composite material according to claim 39, wherein said first set of elongate filaments comprises material selected from the group consisting of: nitinol, titanium, titanium-vanadium-aluminum alloy, cobalt-chromium alloy, cobalt-chromium-molybdenum alloy, cobalt-nickel-chromium-molybdenum alloy, biocompatible stainless steel, tantalum, niobium, hafnium, tungsten.

41. The composite material according to claim 39, wherein said second set of elongate filaments comprise material selected from the group consisting of: poly(ether, ester, ketone), poly(aryl ether), poly(aryl ether ketone), polyethers, polyanhydrides, polyolefins, polyacylate, polymethacrylate, polymethylmethacrylate, polyesters, and copolymers and blends thereof.

42. The composite material according to claim 39, wherein said rigid matrix comprises material selected from the group consisting of: poly(amino acids), polyanhydrides, polycaprolactones, polylactates, polyglycolates, poly(lactic-glycolic acid), polyorthoesters, hydroxyapatite carbonate, corraline, calcium phosphate, tricalcium phosphatem, and blends thereof.

43. The composite material according to claim 39, wherein said rigid matrix is provided to have an initial mass and *in vivo* biodegrades to less than or equal to about one-half of its initial mass within about one year.

44. The composite material according to claim 39, wherein said rigid matrix is provided to have an initial mass and *in vivo* retains more than about one-half of its initial mass for more than one year.

45. The composite material of claim 39, wherein the first set of elongate filaments are composed of a biodegradable material.

46. The composite material of claim 45 wherein first set of elongate filaments exhibit a second degradation rate *in vivo* that is different from the first degradation rate of the matrix.

47. The composite material of claim 39, further including a non-biodegradable structure selected from the group of structures including: eyelets, loops, hooks, bone fasteners, pins, pegs, cements, glues, and combinations thereof.

48. The composite material of claim 39 wherein the rigid matrix is bonded to the first set of elongate filaments or the second set of elongate filaments or both with a biocompatible adhesive.

49. The composite material of claim 39 wherein the rigid matrix is secured to the first set of elongate filaments or the second set of elongate filaments or both via a mechanical interlock.

50. A composite material for use in orthopedic devices comprising:
a first set of filaments comprised of a bio-stable material;
a second set of filaments comprised of a biodegradable material, wherein said first and said second set of filaments are uniformly woven together to provide a flat mesh or a three dimensional network.

51. A composite material for use in orthopedic devices, comprising:
a first sheet composed of a bio-stable material; and
a second sheet composed of a biodegradable material, wherein said first sheet is engaged with said second sheet, and said composite material restricts the motion, in at least one direction, of bone portions fixedly secured to said orthopedic device incorporating said composite material both before and after said second sheet biodegrades.

52. A composite material for use in orthopedic devices, comprising:
a first sheet comprised of a biodegradable material;
a second sheet comprised of a bio-stable material; and
a structure for fixedly attaching said composite material to bone portions, wherein said structure is selected from the group of structures including, eyelets, loops, hooks, bone fasteners, pins, pegs, cements, glues, and combinations thereof.

53. The composite material according to claim 52, wherein said first sheet is attached parallel to the elongate axis of said bone portions, and said second sheet is attached perpendicularly to the elongate axis of said bone portions when said orthopedic device comprising said composite material is attached to said bone portions.

54. The composite material according to claim 52, further including:
a connecting component comprised of a biodegradable material, wherein said connecting component secures said first sheet to said second sheet, and said connecting component is selected from the group consisting of cords, filament, ties, staples, rivets, nut and bolt assemblies, glues, cements, and combinations thereof.

55. A composite material for use in orthopedic devices, said composite material comprising:
a flexible cord comprising a core including a non-biodegradable material; and
a rigid matrix comprising a biodegradable material surrounding said core wherein said rigid matrix restricts the flexibility of said cord.

56. The composition of claim 55 wherein the cord consists of a single filament or solid core.

57. The composite material of claim 55, comprising a plurality of cords.

58. The composite material of claim 57 wherein the plurality of cords are braided.

59. The composite material of claim 57 wherein the plurality of cords are wound together.

60. The composite material according to claim 55 wherein said cord comprises a plurality of elongate filaments woven together to provide a flat mesh or three dimensional network of filaments.

61. The composite material according to claim 55, wherein said non-biodegradable material comprises material selected from the group consisting of:
poly(ether, ether, ketone), poly(aryl ether), poly(aryl ether ketone) polyethers, polyanhydrides, polyolefins, polyacylate, polymethacrylate, polymethylmethacrylate, polyesters, and copolymers and blends thereof.

62. The composite material according to claim 55, wherein said biodegradable material comprises material selected from the group consisting of: poly(amino acids), polyanhydrides, polycaprolactones, polylactates, polyglycolates, poly(lactic-glycolic acid), polyorthoesters, hydroxyapatite carbonate, corraline, calcium phosphate, tricalcium phosphatem, and blends thereof.

63. The composite material according to claim 55, wherein said biodegradable material is provided to have an initial mass and *in vivo* biodegrades to less than or equal to about half its initial mass within about one year.

64. The composite material according to claim 55, wherein said biodegradable material is provided to have an initial mass and *in vivo* retains more than about half its initial mass for more than one year.

65. The composite material of claim 55, including a bone growth material wherein said rigid matrix is impregnated with said bone growth material to promote arthrodesis.

66. The composite material of claim 55, including a bone growth material wherein said cord is impregnated with said bone growth material to promote arthrodesis.

67. The composite material of claim 55, wherein said cord further includes a non-biodegradable structure for fixedly securing said orthopedic devices incorporating said composite material to portions of bone, wherein said structure is selected from the group of structures including, eyelets, loops, hooks, bone fasteners, pins, pegs, cements, glues, and combinations thereof.

68. The composite material of claim 55, wherein said orthopedic device is attached to bone portions and at least some of the load on said orthopedic device is transferred to bone portions as said rigid matrix biodegrades.

69. The composite material of claim 55, wherein said orthopedic devices are attached to bone portions, and said orthopedic devices allow restricted translational, or rotational, or translational and rotational movement of said bone portions after said rigid matrix biodegrades.

70. The composite material of claim 55, wherein the cord has an exterior surface that that is roughened pitted, grooved or knurled.

71. The composite material according to claim 55, wherein the matrix is secured to the cord via a mechanical interlock.

72. A composite material for use in orthopedic devices, said composite material comprising:
a flexible cord comprising a first biodegradable material wherein the flexible cord *in vivo* degrades within a first period of time; and
a rigid matrix comprising a second biodegradable material in contact with the cord, the rigid matrix restricting the flexibility of the cord, wherein matrix *in vivo* degrades within a second period of time different from the first period of time.

73. The composite material according to claim 72 wherein the first biodegradable material comprises is selected from the group consisting of: poly(amino acids), polyanhydrides, polycaprolactones, polylactates, polyglycolates, poly(lactic-glycolic acid), polyorthoesters, hydroxyapatite carbonate, corraline, calcium phosphate, tricalcium phosphatem, and blends thereof.

74. The composite material according to claim 73 wherein the second biodegradable material is selected from the group consisting of: poly(amino acids), polyanhydrides, polycaprolactones, polylactates, polyglycolates, poly(lactic-glycolic acid), polyorthoesters, hydroxyapatite carbonate, corraline, calcium phosphate, tricalcium phosphatem, and blends thereof, and different from the first biodegradable material.

75. The composite material according to claim 73 wherein the cord defines a core and the matrix material surrounds the core.

76. The composite material according to claim 73 comprising a plurality of flexible cords.

77. The composite material according to claim 76 wherein the plurality of flexible cords are braided together.

78. The composite material according to claim 76 wherein the plurality of flexible cords are wound together.

79. The composite material according to claim 76 wherein the flexible cord comprising a first set of flexible filaments and a second set of flexible filaments, wherein the first set of flexible filaments and the second set of flexible filaments are woven together to provide a flat mesh or a three dimensional network of filaments.

80. The composite material of claim 79 wherein the first set of flexible filaments comprise a first biodegradable material and the second set of flexible filaments comprise a second biodegradable material different from the first material.

81. The composite material of claim 80 wherein *in vivo* the first set of flexible filaments degrade within a third period of time and the second set of flexible filaments degrade within a fourth period of time different from the third period of time.

82. The composite material of claim 79 comprising a third set of flexible filaments braided or woven with the first and second sets of flexible cords and defining a second matrix.

83. The composite material of claim 73 wherein the matrix material is bonded to the cord with an adhesive.

84. The composite material of claim 73 wherein the cord has an exterior surface that that is roughened pitted, grooved or knurled.

85. The composite material of claim 73 wherein the matrix material contacts the cord with a mechanical interlocking bond.

86. The composite material according to claim 73 wherein the cord has an exterior surface that has been treated to promote adhesion to the matrix.
